Europäisches Patentamt

European Patent Office

Office européen des brevets

(19)

(11) Veröffentlichungsnummer: **0 197 441**
**B1**

(12) # EUROPÄISCHE PATENTSCHRIFT

(45) Veröffentlichungstag der Patentschrift: **06.02.91**

(51) Int. Cl.⁵: **A 61 L 27/00, A 61 F 2/30**

(21) Anmeldenummer: **86104198.6**

(22) Anmeldetag: **26.03.86**

(54) **Verfahren zur Behandlung von Knochenersatz-Implantaten.**

(30) Priorität: **30.03.85 DE 3511779**
**05.07.85 DE 3524020**

(43) Veröffentlichungstag der Anmeldung:
**15.10.86 Patentblatt 86/42**

(45) Bekanntmachung des Hinweises auf die
Patenterteilung:
**06.02.91 Patentblatt 91/06**

(84) Benannte Vertragsstaaten:
**CH DE FR GB IT LI**

(56) Entgegenhaltungen:
**AT-B- 349 620**
**DE-A-2 138 146**
**DE-A-2 621 122**
**DE-A-3 243 861**

(73) Patentinhaber: **MAN Technologie**
**Aktiengesellschaft**
**Dachauer Strasse 667**
**D-8000 München 50 (DE)**

(72) Erfinder: **Heissler, Herbert, Dr.**
**Rübezahlstrasse 94**
**D-8000 München 83 (DE)**
Erfinder: **Scheer, Wolfgang, Dr. rer. nat.**
**Ignaz-Günther-Strasse 9**
**D-8150 Holzkirchen (DE)**
Erfinder: **Lechner, Fritz, Prof. Dr. med.**
**Klarweinstrasse**
**D-8100 Garmisch-Partenkirchen (DE)**
Erfinder: **Ascherl, Rudolf, Dr.**
**Rathausstrasse 1**
**D-8100 Garmisch-Partenkirchen (DE)**
Erfinder: **Siebels, Wolfgang**
**Ainmillerstrasse 5**
**D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung bezieht sich auf ein Verfahren zur Herstellung von Knochenersatz-Implantaten aus faserverstärktem Kunststoff.

Ziel der Entwicklung von Knochenersatz-Implantaten ist, Patienten während deren Lebensdauer eine sichere und funktionsfähige Unterstützung für Knochenteile leisten zu können, die deren natürliche Aufgaben nicht mehr erfüllen können.

Wie bisher bekannt, werden Implantate mittels eines biokompatiblen Zementes in der entsprechenden Knochenaushöhlung einzementiert. Es hat sich jedoch gezeigt, daß aufgrund der hohen Belastungen ein derart behandeltes Implantat mit der Zeit sich doch aus der Verankerung lockert.

Der Erfindung liegt die Aufgabe zugrunde, ein Verfahren der eingangs genannten Art zu schaffen, mit dem eine längere Lebensdauer von eingesetzten Implantaten erreicht werden kann.

Die Aufgabe wird erfindungsgemäß mit den im Anspruch 1 oder 2 gekennzeichneten Merkmalen gelöst.

Versuche haben gezeigt, daß die freigelegten Fasern eine bessere Voraussetzung für das Verwachsen des Knochens um das Implantat schaffen. Dabei wird gleichzeitig die Verankerung auch dadurch gesichert, daß keine Zementzwischenschicht verwendet wird, die durch Ausbröckeln einen Spalt zwischen Knochen und Implantat herbeiführen kann.

Die Lösung gemäß dem Anspruch 2 hat dazu den Vorteil, daß aufgrund der Faserorientierungen eine gute Druck- und Torsionssteifigkeit erreicht wird, die hohe Belastungen aufnehmen und damit etwaigen Beeinträchtigungen gegen das Anwachsen der Prothese im Knochen entgegenwirken kann. Dieses ist besonders bei Prothesenschäften von Gelenkimplantaten von Bedeutung. Diese Lösung zeichnet sich dadurch aus, daß der vorstehende Vorteil mit fertigungstechnisch einfachen Mitteln erreichbar ist, indem das Implantat aus der Kombination von zwei Teilen mit unterschiedlichen Faserorientierungen hergestellt wird. Der Grundkörper wird vorzugsweise aus unidirektional in Druckrichtung gerichteten Fasern hergestellt, während die Torsionssteifigkeit sich durch das getrennt hergestellte Gewebe ergibt, dessen Fasern am Implantat die für die Torsionssteifigkeit erforderlichen Kreuzlagen bilden.

Zur Förderung der Verwachseigenschaften werden vorzugsweise Kohlenstoffasern verwendet, die in einer Matrix aus Polyphenolglycidylether und 4.4.'-Diaminodiphenylsulfon eingebettet sind. Diese Materialien haben, wie Experimente und Versuche gezeigt haben, eine gute Körperverträglichkeit, so daß das Implantat aufgrund der Materialien und der Oberflächenbeschaffenheit direkt vom Knochengewebe umschlossen werden kann und dieses Wachstum fördert Dieses geschieht, ohne Zwischenschichten verwenden zu müssen, die nur durch eine eventuelle Ablösung vom Implantat selber eine Unsicherheit einbringen.

Bei insbesondere im Preßverfahren hergestellten Implantaten wird vorgeschlagen, das Implantat nach dem Aushärten des Matrixmaterials zur Freilegung der Fasern mechanisch zu behandeln, z.B. durch Aufrauhen.

Wird das Implantat so hergestellt, daß die Formgebung durch eine mechanische Behandlung erfolgt, so kann diese Behandlung gleich so durchgeführt werden, daß sie gleichzeitig die Fasern an der Oberfläche freilegt, wie z.B. durch Fräsen.

Das Gewebe wird vorzugsweise auch aus Kohlenstoffasern hergestellt und in Polyphenolglycidylether mit 4.4.'-Diaminodyphenylsulfon getränkt und anschließend zur Feilegung der Fasern oberflächlich aufgerauht.

Eine bevorzugte Anwendung des erfindungsgemäßen Verfahrens ist eine Gelenkprothese, bestehend aus einem Schaft mit einer daran verbundenen Gelenkkugel, die mit einer Gelenkpfanne zusammenwirkt. Bei einer derartigen Prothese ist der in den Knochen einzuführende Schaftteil sowie die Außenoberfläche der Gelenkpfanne faserfreilegend zu behandeln.

Damit die durch die Erfindung erreichbaren, die Lebensdauer einer Gelenkprothese verlängernden guten Verwachseigenschaften nicht durch "Schwachstellen" gemindert werden, wird vorgeschlagen, den Prothesenschaft mit einem Stützkragen am vom Knochen herausragenden Teil sowie einer Gelenkkugel aus Keramik, beispielsweise Aluminiumoxid, Siliziumnitrid zu versehen. Die Gelenkpfanne sollte dabei ein Gewinde aufweisen, um der Pfanne durch Einschrauben eine sichere Verankerung zu geben. Erst diese Merkmale werden in Verbindung mit der Oberflächenbehandlung die Dauerhaftigkeit eines Implantates für Gelenke gewährleisten.

Durch den Stützkragen des Schaftes wird eine Axialverschiebung bzw. Reibung weitgehend vermieden, um so den Verwachsprozeß möglichst nicht zu stören. Ferner ist für die Lebensdauer eines Gelenkimplantates die Gelenkstelle selber von großer Bedeutung. Auch diese sollte die zu erwartende Lebensdauer der Verankerungen nicht schmälern. Es hat sich gezeigt, daß die Paarung Keramikkugel mit einer Pfanne aus dem oben genannten kohlenstoffaserverstärkten Verbundwerkstoff keinen nennenswerten Abrieb aufzeigt. Für den Schaft kann das Gewebe in Form eines Schlauches hergestellt und über den Schaft gezogen werden, wobei der Schlauch am dickeren Ende des Schaftes stark gedehnt wird, so daß die Fasern eine erwünschte straffe Lage einnehmen, wie sie sonst nur im Wickelverfahren erreichbar ist.

Das Verfahren wird anhand eines in der Zeichnung schemattisch dargestellten Ausführungsbeispieles näher erläutert.

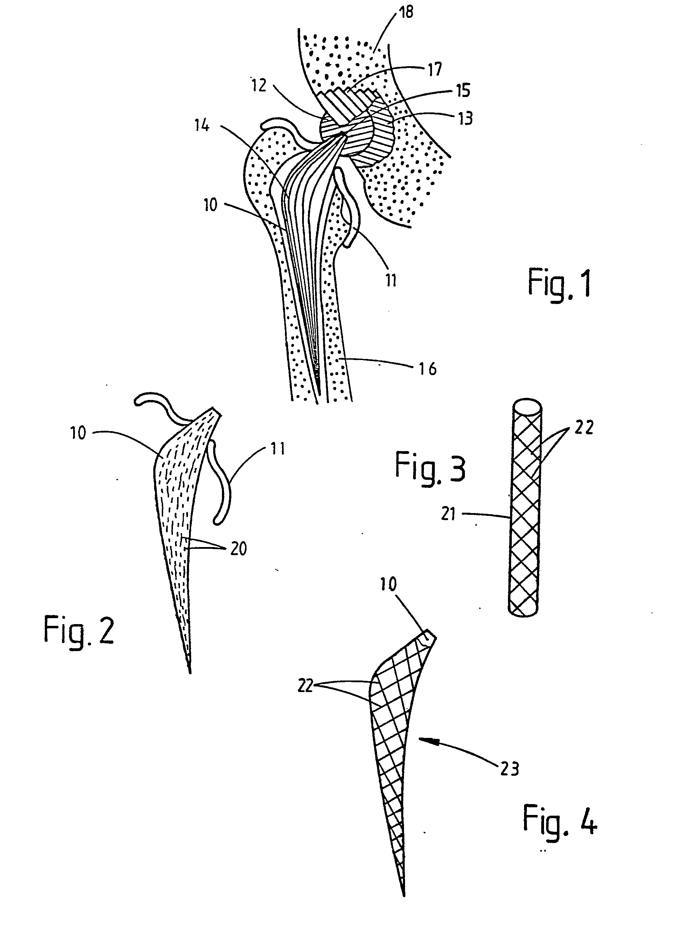

Fig. 1 zeigt ein Hüftgelenk-Implantat, bestehend aus einem Schaft 10 mit einem Stützkragen 11 und einer Gelenkkugel 12 sowie aus einer Gelenkpfanne 13. In dem kohlenstoffaserverstärkten Schaft sind die Fasern 14 unidirektional und annähernd parallel zur Außenkontur des Schaftes 10 gerichtet, um diesen Prothesenteil bei der vorwiegend in axialer Richtung auferlegten Belastung die notwendige Steifigkeit zu verleihen.

Das obere Ende 15 des Schaftes 10 ist zur Aufnahme der Gelenkkugel 12 konisch ausgebildet. Die Kugel 12 ist auf dem Konus 15 aufgesteckt.

Um eine optimierte Gleiteigenschaft zwischen dem Gelenkkopf 12 und Gelenkpfanne 13 zu erhalten, ist der Gelenkkopf 12 aus Keramik und die Gelenkpfanne 13 aus einem kohlenstoffaserverstärktem Werkstoff mit einer Matrix aus Polyphenolglycidylether und 4.4.'-Diaminodiphenylsulfon hergestellt.

Um eine Verschiebung des Schaftes 10 innerhalb des Femurschaftes 16 bei den axialen Belastungen möglichst gering zu halten, ist der Stützkragen 11 so am Schaft 10 angeordnet, daß er auf das obere Ende des Femurknochens 16 zu liegen kommt. Für einfeste Verankerung der Gelenkpfanne 13 sorgt ein Gewinde 17, über das die Gelenkpfanne 13 in den Beckenknochen 18 einschraubbar ist.

Die bisher beschriebene Ausgestaltung der Prothese dient zur festen und möglichst dauerhaften Verankerung derselben im Knochen durch die genannten Eigenschaften der Prothese selber, was allerdings noch nicht ausreicht, um eine sichere und funktionsfähige Unterstützung auf Lebzeiten des Patienten sicherzustellen. Wichtig ist nämlich außerdem, daß diese Fremdkörper nicht vom menschlichen Organismus bzw. dem Knochen abgestoßen werden. Für die gute Verträglichkeit sorgt die Materialauswahl, während zur Förderung des Verwachsens des Knochens um das Implantat eine äußere Behandlung der Kontaktflächen des Implantates mit dem Knochen sorgt. Diese Behandlung besteht darin, den Schaft unterhalb des Stützkragens 11 oberflächlich mechanisch so aufzurauhen, daß, wie in Fig. 2 gezeigt, die Fasern 20 freigelegt werden. Diese Fasern ermöglichen durch die gute Körperverträglichkeit des Kohlenstoffes eine anfängliche Verbindung mit dem Knochenmaterial, so daß dadurch ein verbessertes Verwachsen des Knochens um den Schaft 10 möglich ist.

Das Freilegen von Fasern und Faserspitzen 20 kann auf jede Art geschehen. So erfolgt die Freilegung z.B. der Gelenkpfanne 13 direkt in Verbindung mit dem Fräsen des Gewindes 17.

Die freigelegten, körperverträglichen Kohlenstoffasern 20 fördern in einem beachtlichem Maße das Verwachsen des Knochenmaterials um das Implantat. Dieser Verwachsvorgang kann durch eine möglichst geringe relative Beweglichkeit zwischen Prothesenteil und Knochen unterstützt werden. Dieses wird erreicht durch die Kombination der folgenden Merkmale; die hohe Steifigkeit aufgrund der gewählten Fasern aus Kohlenstoff und deren Orientierung im Schaft 10, die Abstützung durch den Stützkragen 11 des Schaftes 10, der beibehaltbare geringe Spalt zwischen Gelenkkugel 12 und Gelenkpfanne 13 aufgrund der guten Gleiteigenschaften, sowie die Schraubverankerung der Gelenkpfanne 13.

Wenn das Implantat 10 in einer fertigungstechnisch einfachen Art nur mit unidirektionalen Fasern 14 hergestellt wird, kann eine Faserlage mit anders gerichteten Fasern dadurch aufgebracht werden, daß ein Gewebe, vorzugsweise ein Gewebeschlauch 21 getrennt hergestellt, in einem Matrixmaterial getränkt und auf den Implantatgrundkörper 10 aufgezogen wird. Das Gewebe 21 wird so aufgebracht, daß dessen Fasern schräg zur Faserrichtung des Grundkörpers 10 verlaufen derart, daß die Fasern 14 und 22 des Verbundes 23 im wesentlichen entsprechend den Beanspruchungsrichtungen gerichtet sind.

Für den Grundkörper 10 verlaufen die Fasern 22 des Gewebeschlauches 21 schräg zur Schlauchlängsachse so, daß der Schlauch dehnbar ist. Beim Aufbringen, des Schlauches 21 auf den Grundkörper 10 nehmen die Fasern 22 im dickeren Bereich des Grundkörpers, in dem der Schlauch gedehnt wird, etwa die gewünschte Orientierung von 45° ein. Zur Spitze hin verringert sich dieser Winkel bis auf etwa 15° bis 20°.

**Patentansprüche**

1. Verfahren zur Herstellung von Knochenersatz-Implantaten aus faserverstärktem Kunststoff, dadurch gekennzeichnet, daß nach der Herstellung des faserverstärkten Kunststoffkörpers (10) die Oberfläche mindstens teilweise so behandelt wird, daß die Fasern (20) zumindest teilweise freigelegt werden.

2. Verfahren nach Anspruch 1, dadurch gekennzeichnet, daß der faserverstärkte Kunststoffkörper aus einem Grundkörper (10) mit annähernd nur in einer Richtung verlaufenden Fasern (14) hergestellt wird, daß der Grundkörper mit einem aus Fasern oder Einzelfilamenten (22) hergestelltes und mit Matrixmaterial getränktes Gewebe (21) umspannt wird derart, daß sich die Fasern des Gewebes mit denen des Grundkörpers kreuzen, daß anschließend der Verbund aus Grundkörper und Gewebe zur Formgebung verpreßt wird.

3. Verfahren nach Anspruch 1 oder 2, dadurch gekennzeichnet, daß für das Implantat bzw. den Grundkörper Polyphenolglycidylether und 4.4.' Diaminodiphenylsulfon eingebettete Kohlenstofffasern verwendet werden.

4. Verfahren nach Anspruch 2, dadurch gekennzeichnet, daß für das Gewebe (21) das gleiche Material wie für den Grundkörper (10) verwendet wird.

5. Verfahren nach einem der vorhergehenden Ansprüche, dadurch gekennzeichnet, daß die Oberfläche mechanisch, insbesondere durch Aufrauhen behandelt wird.

6. Verfahren nach einem der Ansprüche 1 bis 4, dadurch gekennzeichnet, daß mit der faserfreigelegten Behandlung gleichzeitig die Formgebung des Implantats erfolgt.

7. Verfahren nach Anspruch 6, dadurch gekennzeichnet, daß die Implantatoberfläche gefräst wird.

8. Verfahren nach einem der vorhergehenden Ansprüche, dadurch gekennzeichnet, daß bei Anwendung des Verfahrens bei einer Gelenkendoprothese mit einem Schaft, einer daran befestigten Gelenkkugel sowie einer Gelenkpfanne (13) die Fasern (14) des Schaftes (10) unidirektional und annähernd parallel zur Schaftoberfläche gelegt werden, daß der Schaft mit einem Stützkragen (11) als Knochenauflage versehen wird, und daß an der Außenoberfläche der Gelenkpfanne (13) ein Gewinde (17) eingearbeitet wird, wobei zumindest die Fasern an der Oberfläche des Schaftes unterhalb des Stützkragens und des Gewindeteiles der Gelenkpfanne freigelegt werden.

9. Verfahren nach Anspruch 8, dadurch gekennzeichnet, daß der Schaft (10) und die Gelenkpfanne (13) aus kohlenstoffaserverstärktem Kunststoff und die Gelenkkugel aus Keramik hergestellt werden.

10. Verfahren nach Anspruch 2 und 9, dadurch gekennzeichnet, daß der Schaft (10) mit dem Gewebe (21) umspannt wird.

**Revendications**

1. Procédé de fabrication d'un implant de prothèse osseuse en matière synthétique renforcée par des fibres, procédé caractérisé en ce qu'après avoir réalisé le corps en matière synthétique (10) renforcé de fibres, on traite la surface, au moins partiellement pour libérer au moins partiellement les fibres (20).

2. Procédé selon la revendication 1, caractérisé en ce que le corps de matière synthétique renforcé par des fibres est composé d'un corps de base (10) avec des fibres (14) dirigées pratiquement selon une direction, en ce que l'on réalise le corps de base avec des fibres ou un seul filament (22) et on imprègne le tissu (21) à l'état détendu, avec un matériau formant matrice de manière que les fibres du tissu se croisent avec celles du corps de base et ensuite on presse l'élément composite formé du corps de base et du tissu pour le mettre en forme.

3. Procédé selon la revendication 1 ou 2, caractérisé en ce que pour l'implant ou le corps de base, on utilise du polyphénolglycidyléter et du 4,4'-diaminodiphénylsulfone chargés de fibres de carbone.

4. Procédé selon la revendication 2, caractérisé en ce que pour le tissu (21), on utilise le même matériau que pour le corps de base (10).

5. Procédé selon l'une des revendications précédentes, caractérisé en ce qu'on traite mécaniquement la surface en particulier pour la rendre rugueuse.

6. Procédé selon l'une des revendications 1 à 4, caractérisé en ce qu'on réalise la mise en forme de l'implant en même temps que l'on traite les fibres libérées.

7. Procédé selon la revendication 6, caractérisé en ce qu'on fraise la surface de l'implant.

8. Procédé selon l'une quelconque des revendications précédentes, caractérisé en ce que par l'application du procédé à une prothèse de hanche avec une tige à laquelle est fixée une rotule ainsi qu'une coupelle (13), on dispose les fibres (14) de la tige (10) de manière unidirectionnelle et sensiblement parallèle à la surface de la tige, on munit la tige d'un collet d'appui (11) servant d'appui osseux et en ce qu'on usine la surface extérieure de la coupelle (13) pour réaliser un filetage (17) et on libère au moins les fibres à la surface de la tige sous le collet d'appui et de la partie filetée de la coupelle.

9. Procédé selon la revendication 8, caractérisé en ce qu'on réalise la tige (10) et la coupelle (13) en matière synthétique renforcée par de la fibre de carbone et on réalise la rotule en matière céramique.

10. Procédé selon la revendication 2 ou 9, caractérisé en ce qu'on tend du tissu (21) sur la tige (10).

**Claims**

1. A process for the manufacture of bone-replacement implants made from a fibre-reinforced synthetic-material, characterized in that after manufacture of the fibre-reinforced synthetic-body (10) the surface is at least partially treated in such a manner that the fibres (20) are at least partially exposed.

2. A process in accordance with Claim 1, characterized in that the fibre-reinforced synthetic-body is manufactured from a basic body (10) with fibres (14) running approximately in one direction only, in that the basic body is enclosed, by means of a fabric (21) manufactured from fibres or individual filaments (22) and impregnated with a matrix material, in such a manner that the fibres of the fabric cross those of the basic body, in that the composite of basic body and fabric is subsequently pressed for the purposes of shaping.

3. A process in accordance with Claim 1 or 2, characterized in that polyphenolglycidylether- and 4.4.'-diaminodiphenylsulfon-embedded carbon fibres are used for the implant and/or the basic body.

4. A process in accordance with Claim 2, characterized in that the same material is used for the fabric (21) as for the basic body (10).

5. A process in accordance with any one of the preceding Claims, characterized in that the surface is mechanically treated, in particular by means of roughening.

6. A process in accordance with any one of Claims 1 to 4, characterized in that the shaping of the implant takes place at the same time as the fibre-exposing treatment.

7. A process in accordance with Claim 6, characterized in that the implant surface is milled.

8. A process in accordance with any one of the preceding Claims, characterized in that when using the process with a joint endoprosthesis with a shaft, a condyle fastened thereto and a joint socket (13), the fibres (14) of the shaft (10) are placed unidirectionally and approximately parallel to the shaft surface, in that the shaft has a supporting collar (11) as a bone rest, and in that a thread (17) is incorporated on the outer surface of the joint socket (13), at least the fibres which are on the surface of the shaft and below the supporting collar and the threaded part of the joint socket being exposed.

9. A process in accordance with Claim 8, characterized in that the shaft (10) and the joint socket (13) are manufactured from a synthetic material reinforced with carbon fibres and the condyle is manufactured from ceramics.

10. A process in accordance with Claim 2 and 9, characterized in that the shaft (10) is enclosed by the fabric (21).

Fig. 1

Fig. 2

Fig. 3

Fig. 4